Europäisches Patentamt

European Patent Office

Office européen des brevets

(19)

(11) Publication number: **0 162 725 B1**

(12) **EUROPEAN PATENT SPECIFICATION**

(45) Date of publication of patent specification: **15.04.92**    (51) Int. Cl.[5]: **C12N 15/74**

(21) Application number: **85303682.0**

(22) Date of filing: **24.05.85**

(54) Chimeric plasmid vector.

(30) Priority: **24.05.84 JP 105411/84**

(43) Date of publication of application:
**27.11.85 Bulletin 85/48**

(45) Publication of the grant of the patent:
**15.04.92 Bulletin 92/16**

(84) Designated Contracting States:
**AT BE CH DE FR GB IT LI LU NL SE**

(56) References cited:

**CHEMICAL ABSTRACTS, vol. 99, no. 17, 24th October 1983, page 138, abstract no. 134527e, Columbus, Ohio, US; J.B. PERKINS et al.: "Streptococcus plasmid pAMalpha1 is a composite of two separable replicons, one of which is closely related to Bacillus plasmid pBC16" & J. BACTERIOL. 1983, 155(2), 607-15**

**JOURNAL OF BACTERIOLOGY, vol. 140, November 1979, pages 400-407; GYNHEUNG AN et al.: "Plasmid vehicles for direct cloning of escherichia coli promoters"**

**NUCLEIC ACIDS RESEARCH, vol. 11, no. 6, 25th March 1983, pages 1645-1654, IRL Press**

**Ltd., Oxford, GB; L. DENTE et al.: "pEMBL: a new family of single stranded plasmids"**

(73) Proprietor: **KABUSHIKI KAISHA YAKULT HONSHA**
**1-19, Higashishinbashi 1-chome**
**Minato-ku Tokyo 105(JP)**

(72) Inventor: **Ishiwa, Hiromi**
**1715-7, Josuihon-cho Kodaira**
**Tokyo(JP)**
Inventor: **Shibahara, Harue**
**1032-7, Itsukaichi Nishitama**
**Tokyo(JP)**
Inventor: **Mutai, Masahiko**
**988, 4-chome Shimizu Higashiyamato**
**Tokyo(JP)**

(74) Representative: **Brewer, Leonard Stuart et al**
**SANDERSON & CO. European Patent Attorneys 34, East Stockwell Street**
**Colchester Essex CO1 1ST(GB)**

## Description

The present invention relates to a novel chimeric plasmid vector which is useful as a cloning vector for Escherichia coli or Bacillus subtilis.

## BACKGROUND OF THE INVENTION

In genetic manipulation in vitro, the genetic information carried on a desired foreign DNA is expressed by introducing the DNA into a host cell and, for this purpose, it is important to employ a vector optimum for the host cell. Of the vectors presently known as being operable for genetic manipulation, those which form host-vector systems using E. coli as the host are the most typical of the achievements of the past research and development efforts. Efforts are however also being made for the research and development of host-vector systems using microorganisms other than E. coli. These microorganisms include B. subtilis which is a microorganism important for industrial use, Streptomyces which are important sources of various antibiotics, and yeasts which are used broadly in the field of brewing technology.

In order that a vector be operable as such, it must at least contain a sequence essential for self-replication and have a recognition and cleavage site for a restriction enzyme at which a donor DNA can be inserted within the vector, as well known in the art. From the practical point of view, however, vectors to be used for purposes of genetic manipulation are required to meet various other serious requirements. These requirements include the availability of restriction enzymes which will contribute to and facilitate the genetic manipulation, the variety of option for the recognition and cleavage sites available in the restriction enzymes, high efficiencies in the transformation and its phenotypic expression, the presence of marker genes useful for the detection of, for example, the transformants, the compatibility of the vectors with the host cells, the broadness of the range of the host cells compatible with the vectors, the stability of the vectors in the host cells, and the adaptability of the vectors to possible biological containment. By reason of these numerous strict requirements, there have been developed actually only a limited number of useful vectors even in host-vector systems using E. coli and B. subtilis.

Under the circumstances, Applicants have made extensive research and development efforts in quest of useful plasmid vectors, with a view to providing established host-vector systems using such vectors. These efforts have been directed particularly to host-vector systems using E. coli, the microbial properties of which are best known among microorganisms of interest, and B. subtilis.which is useful for industrial purposes as a microorganism productive of, typically, amylase. The efforts have led to the discovery that certain derivatives of a chimeric plasmid vector synthesized from the plasmid pAMα1 of Streptococcus faecalis and the vector pACYC177 of E. coli have various properties which are favourable in vectors for use in genetic manipulation of E. coli and B. subtilis. These chimeric plasmid vectors are disclosed in Applicants' copending United States Patent Application Serial No. 574,180 (which corresponds to EPC Application No. 84-30-0403.7 and Japanese Patent Applications Nos. 58-9740, 58-103196, 58-159345 and 59-2361) and Japanese Patent Application No. 58-9739.

Applicants have further made efforts to develop more and other useful plasmid vectors. These continued efforts have now resulted in the development of excellent chimeric shuttle vectors which not only have beneficial microbial properties comparable to those of known plasmid vectors but also permit the use of various restriction enzymes. The present invention is concerned particularly with these novel and useful chimeric plasmid vectors.

## SUMMARY OF THE INVENTION

In accordance with the present invention, there is provided a chimeric plasmid vector containing (a) a tetracycline resistance gene region (Tc) derived from the plasmid pAMα1 of Streptococcus faecalis DS5 (ATCC14508), (b) an ampicillin resistance gene region (Amp) derived from the vector pACYC177 of E. coli, (c) a first DNA replication origin (OripAMα1) derived from the plasmid pAMα1, (d) a second DNA replication origin (Ori177) derived from the vector pACYC177, and (e) a polylinker region having recognition and cleavage sites for the restriction enzymes EcoRI at one terminal and HindIII at the other terminal of the polylinker DNA sequence. Unique recognition and cleavage sites for the restriction enzymes BalI, HpaI and EcoRV of the plasmid vector are located in the tetracycline resistance gene region, and unique recognition and cleavage sites for the restriction enzymes PvuI, BglI, and BanI of the plasmid vector are located in the ampicillin resistance gene region.

The present chimeric plasmid vector can be constructed by enzymatically cleaving the plasmid pAMα1 of Streptococcus faecalis DS5 and the vector pACYC177 of E. coli so that the tetracycline resistance gene

2

region (Tc) and the first DNA replication origin (OripAMα1) are contained in the DNA fragment derived from the plasmid pAMα1 and the ampicillin resistance gene region (Amp) and the second DNA replication origin (Ori177) are contained in the DNA fragment derived from the vector pACYC177. The two DNA fragments are ligated together with a polylinker region having the recognition and cleavage sites for the restriction enzymes EcoRI and HindIII at the opposite terminals of the polylinker sequence. Examples of the chimeric plasmid vector thus obtained have a molecular weight of about 3.0 megadaltons and are named pHY300PLK, pHY301PLK and PHY302PLK.

Accordingly, the invention provides a process for preparing the present chimeric plasmid vector, which process comprises:

(A) enzymatically cleaving a chimeric plasmid containing:

(a) a tetracycline resistance gene region derived from the plasmid pAMα1 of Streptococcus faecalis,
(b) an ampicillin resistance gene region derived from the vector pACYC177 of Escherichia coli,
(c) a DNA replication origin derived from said plasmid pAMα1, and
(d) a DNA replication origin derived from said vector pACYC177,

said tetracycline resistance gene region containing a unique site at which said chimeric plasmid is recognized and cleaved by the restriction enzyme BalI and said ampicillin resistance gene region containing a unique site at which said chimeric plasmid is recognized and cleaved by the restriction enzymes BglI and PstI,

the enzymatic cleavage being such that the tetracycline resistance gene region (a) and the DNA replication origin (c) are retained in the DNA sequence derived from the plasmid pAMα1, and the ampicillin resistance gene region (b) and the DNA replication origin (d) are retained in the DNA sequence derived from the vector pACYC177; and

(B) ligating to include the polylinker region. The chimeric plasmid starting material, and in particular the plasmid pHY340, are described in the Applicants' European patent application 84-30-0403.7 referred to above. That application should be consulted for further details, and its disclosure is incorporated herein by reference. Between the cleaving stage (A) and the ligating stage (B), the material can be modified as appropriate so that the desired chimeric plasmid vector is obtained.

The invention also provides a process for preparing the present chimeric plasmid vector, which process comprises excising the section of the plasmid pHY336 between the EcoRI and HindIII sites and substituting it by the polylinker region. The plasmid pHY336 can itself be prepared by a process comprising:

(a) partially digesting plasmid pHY340 with EcoRI to obtain a fragment open at one of the two EcoRI sites of the plasmid pHY340, the fragment having a molecular weight of 3.4 megadaltons;
(b) treating the fragment with T4 DNA polymerase and T4 DNA ligase to produce a circular DNA;
(c) introducing the circular DNA into Escherichia coli and extracting plasmid pHY339 from the resultant transformant, the plasmid having unique EcoRI and SacII sites; and
(d) cleaving the plasmid pHY339 with SacII, treating the resultant linear DNA with T4 DNA polymerase, adding phosphorylated HindIII linker, and further treating with T4 DNA ligase, to produce plasmid pHY336 which has a HindIII site in place of the SacII site in the plasmid pHY339.

The invention provides also a method employing a vector to convey a gene into a host cell, usually of a microorganism, especially a bacterium, which may be a gram-positive or gram-negative bacterium, especially Escherichia coli or Bacillus subtilis, in which method the present chimeric plasmid vector is used as vector.

The invention provides also of course the host produced by this method (including the descendants of the immediate host). The invention also provides biosynthesis employing a gene cloned by this host.

The invention also provides a microorganism as deposited at the Fermentation Research Institute, Agency of Industrial Science and Technology, Japan, under the deposition number FERM BP-744 or FERM BP-753.

As discussed above, a chimeric plasmid vector according to the present invention is characterized in that it contains tetracycline and ampicillin resistance gene regions carried on its DNA. It is for this reason capable of conferring resistance to these drugs on the host cell by transformation of E. coli. The plasmid vector is further capable of conferring resistance to tetracycline on the host cell by transformation of B. subtilis. This capability of the chimeric plasmid vector provides a selective marker which is operable, in the process of transformation, for the detection and selection of the transformant, viz., the strain carrying the recombinant plasmid into which the desired foreign DNA has been introduced.

A chimeric plasmid vector according to the present invention is further characterized in that the sole recognition and cleavage sites for the restriction enzymes BalI, HpaI and EcoRV of the plasmid vector are located in the tetracycline resistance gene region, and the sole recognition and cleavage sites for the restriction enzymes PvuI, BglI and BanI of the plasmid vector are located in the ampicillin resistance gene region. Thus, when such a chimeric plasmid vector is to be cleaved with any of these restriction enzymes, the plasmid vector is not cut into numerous fragments at unwanted sites of the DNA. A desired foreign DNA can therefore be inserted into the plasmid vector at a selected one of the particular recognition and cleavage sites of the vector.

A chimeric plasmid vector according to the present invention is further characterized by the presence therein of a polylinker region bearing a considerable number of operable cloning sites, terminal ones of which are identified as the recognition and cleavage sites for the restriction enzymes EcoRI at one terminal and HindIII at the other terminal of the polylinker DNA sequence. This will contribute to further improvement of the utility of the chimeric plasmid vector to be used as a cloning vector.

Furthermore, a chimeric plasmid vector according to the present invention has been found to be capable of replicating stably not only in E. coli but also in B. subtilis due to favorable interaction between the two particular replication origins (OripAMα1, Ori177) derived from the plasmid pAMα1 and vector pACYC177. The chimeric plasmid vector can therefore be used advantageously as a shuttle vector to reciprocate between the hosts of E. coli and B. subtilis and provides substantially equal transformation efficiencies for these host bacteria.

As is well known, the most advanced of the DNA cloning host-vector systems which are presently under research and development is the EK system which consists of Gram-negative E. coli K-12 strain and its plasmid or phage vector. Such a host-vector system is useful for the cloning or expression of certain kinds of genes derived from the Gram-negative strain and certain kinds of genes derived from Gram-positive bacteria such as B. subtilis. When used for the cloning or expression of genes derived from Gram-positive bacteria, the EK host-vector system still has far more problems to be circumvented than those concomitant with the system for the cloning or expression of genes derived from the Gram-negative strain. Various attempts are being thus made to overcome such difficulties attending the host-vector systems used for the cloning or expression of genes derived from Gram-positive bacteria. These attempts centre on the host-vector system using B. subtilis, one of the most useful microorganisms as host bacteria, and involve analysis into the genetic mechanism of B. subtilis and development of cloning systems using the B. subtilis whose microbiological behaviour is very different from that of E. coli. Development of an established host-vector system using B. subtilis is under these circumstances an urgent demand from industry.

As discussed previously, the chimeric plasmid vector provided by the present invention has a relatively small molecular weight of about 3.0 megadaltons and is operable as a shuttle vector capable of reciprocating between E. coli which is typical of the Gram-negative bacteria and B. subtilis which is typical of the Gram-positive bacteria. Such a shuttle vector is expected to provide a step forward to the establishment of a cloning system using B. subtilis as the host bacterium and will thus prove useful for various industrial purposes.

The chimeric plasmid vector according to the present invention is further of importance for its future prospect since the vector may be utilized extensively for the analysis into and the molecular breeding of genes of other kinds of Gram-positive bacteria belonging to, for example, the genus Lactobacillus and the genus Bifidobacterium.

## BRIEF DESCRIPTION OF THE DRAWINGS

The chimeric plasmid vector according to the present invention will be more clearly understood from the following description taken in conjunction with the accompanying drawings in which:

Fig. 1 is a flowchart showing the steps involved in the synthesis of a preferred example, specifically named pHY300PLK, of a chimeric plasmid vector according to the present invention;

Fig. 2 is a cleavage map showing the sequence of recognition and cleavage sites of the chimeric plasmid vector pHY300PLK;

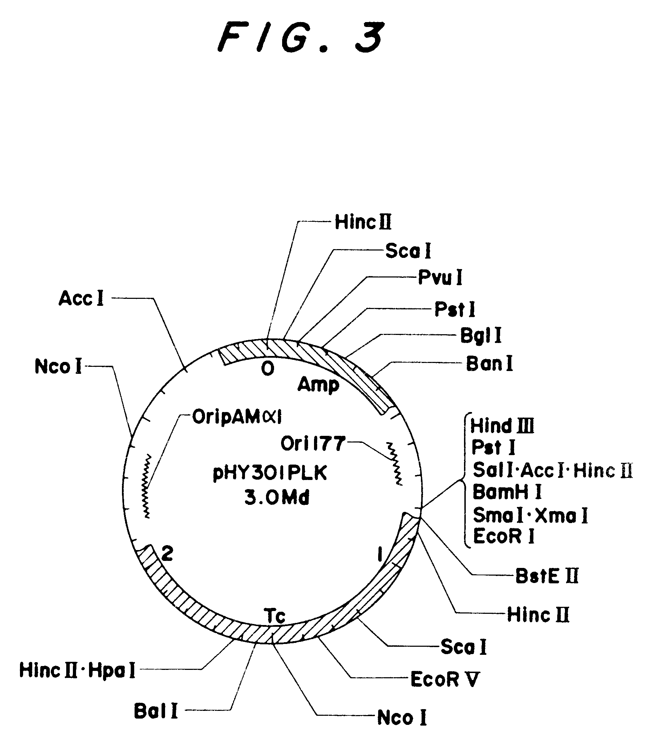

Fig. 3 is a cleavage map showing the sequence of recognition and cleavage sites of another preferred example, named pHY301PLK, of a chimeric plasmid vector according to the present invention; and

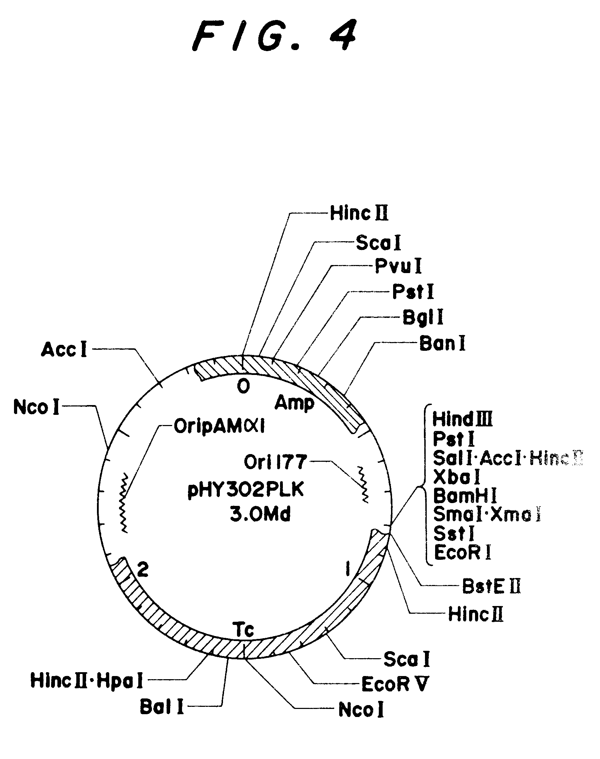

Fig. 4 is a cleavage map showing the sequence of recognition and cleavage sites of still another example, named pHY302PLK, of a chimeric plasmid vector according to the present invention.

## DETAILED DESCRIPTION OF THE INVENTION

Chimeric plasmid pHY340 can be used as follows as a source of a plasmid vector according to the present invention.

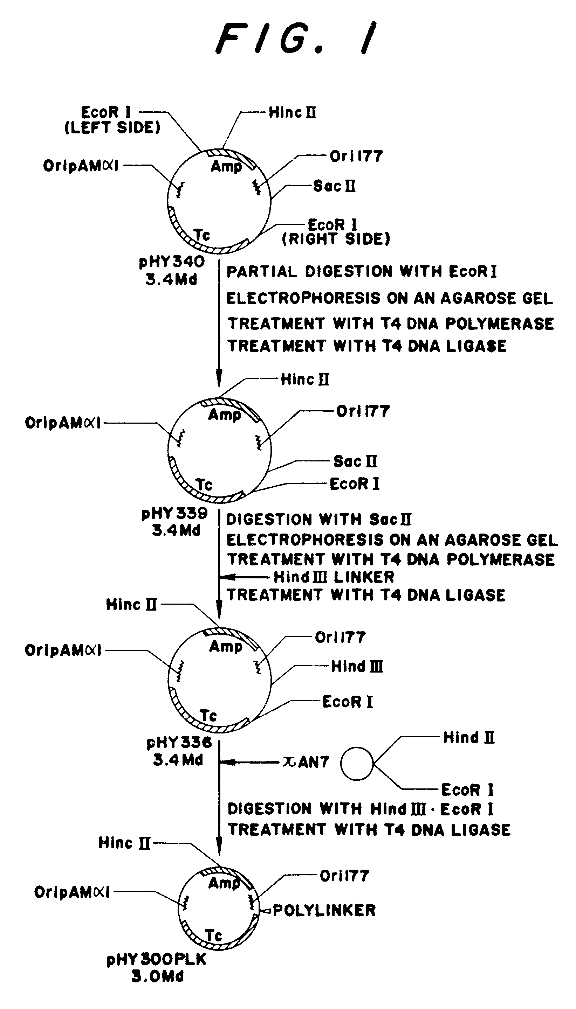

The plasmid pHY340 can be prepared as taught in United States Patent Application Serial No. 574,180 (as well as EPC Application No. 84-30-0403.7 and Japanese Patent Application No. 58-159345). As shown in Fig. 1, the plasmid pHY340 has a molecular weight of about 3.4 megadaltons and contains two EcoRI sites. The circular plasmid is partially digested with EcoRI to obtain fragments each open at one (shown to the left in Fig. 1) of these two EcoRI sites. The resultant DNA fragment is subjected to low-melting-point electrophoresis on an agarose gel, whereupon the DNA having a molecular weight of 3.4 megadaltons is extracted from the gel plate. The linear DNA thus extracted is treated with T4 DNA polymerase and T4 DNA ligase to produce circular DNA. The circular DNA is introduced into E. coli and a plasmid is extracted from the resultant transformant. One of the synthesized plasmids, named pHY339, has the unique EcoRI and SacII sites as shown in Fig. 1 and can be cut at these two sites into two segments, the smaller one of which has a molecular weight of about 0.4 megadalton. The plasmid pHY339 is cleaved with SacII and the resultant linear DNA is treated with T4 DNA polymerase and, upon addition of phosphorylated HindIII linker (dpCAAGCTTG), further treated with T4 DNA ligase, thus producing a plasmid named pHY336 as also shown in Fig. 1. The plasmid pHY336 has a HindIII site in place of the SacII site in the plasmid pHY339. The section of this plasmid pHY336 between the EcoRI and HindIII sites is excised from the plasmid and is substituted by an appropriate polylinker. The polylinker may be a DNA fragment derived from, for example, plasmid πAN7 and having a sequence of recognition and cleavage sites for restriction enzymes sequenced in the form of

$$
\text{EcoRI} - \left\{ \begin{array}{c} \text{SmaI} \\ \hline \text{XmaI} \end{array} \right\} - \text{BamHI} - \left[ \begin{array}{c} \text{SalI} \\ \hline \text{AccI} \\ \hline \text{HincII} \end{array} \right] - \text{PstI} - \text{BglII} - \text{XbaI} - \text{HindIII}.
$$

Specific examples of the polylinker operable in the process of preparing a chimeric plasmid vector according to the present invention include:

1) a DNA fragment having a nucleotide sequence of

5'-GAATTCCCGGGGGATCCGTCGACCTGCAGATCTCTAGAAGCTT-3',

which is characterized by the sequence of recognition and cleavage sites for restriction enzymes sequenced in the form of

$$
\text{EcoRI} - \left[ \begin{array}{c} \text{SmaI} \\ \hline \text{XmaI} \end{array} \right] - \text{BamHI} - \left[ \begin{array}{c} \text{SalI} \\ \hline \text{AccI} \\ \hline \text{HincII} \end{array} \right] - \text{PstI} - \text{BglII} - \text{XbaI} - \text{HindIII},
$$

2) a DNA fragment having a nucleotide sequence of

5'-GAATTCCCGGGGGATCCGTCGACCTGCAGCCAAGCTT-3',

which is characterized by the sequence of recognition and cleavage sites for restriction enzymes sequenced in the form of

$$
\text{EcoRI} - \left\{ \begin{array}{c} \text{SmaI} \\ \hline \text{XmaI} \end{array} \right\} - \text{BamHI} - \left[ \begin{array}{c} \text{SalI} \\ \hline \text{AccI} \\ \hline \text{HincII} \end{array} \right] - \text{PstI} - \text{HindIII}
$$

and
3) a DNA fragment having a nucleotide sequence of

5'-GAATTCGAGCTCGCCCGGGGGATCCTCTAGAGTCGACCTGCAGCCCAAGCTT-3',

which is characterized by the sequence of recognition and cleavage sites for restriction enzymes sequenced in the form of

5

$$\underline{EcoRI} - \underline{SstI} - \left[\begin{array}{c}\underline{SmaI} \\ \overline{\phantom{x}} \\ \underline{XmaI}\end{array}\right] \underline{BamHI} - \underline{XbaI} - \left[\begin{array}{c}\underline{SalI} \\ \underline{AccI} \\ \underline{HincII}\end{array}\right] - \underline{PstI} - \underline{HindIII}.$$

It may however be remembered that these examples of the polylinker operable for the preparation of a chimeric plasmid vector according to the present invention are merely for the purpose of description and are not limitative of the scope of the invention.

The present invention will be better understood from the following Examples.

Example 1

(1) Preparation of Plasmid pHY339 (Removal of one of the EcoRI sites from plasmid pHY340)

Five point seven ul of a 10-fold concentrated buffer containing 100 mM Tris-HCl (pH 7.6), 70mM $MgCl_2$, 70 mM $\beta$-mercaptoethanol and 500 mM NaCl was added to 53 $\mu$l of pHY340 DNA (100 $\mu$g/ml), followed by addition of 2 $\mu$l of 6 unit/$\mu$l EcoRI. The mixture of these was divided into three equal fractions, which were incubated at 37°C for 15, 20 and 25 minutes, respectively, to digest the DNA in each fraction with EcoRI. Each of the fractions was heated at 70°C for 5 minutes to terminate the reaction and was thereafter electrophoresed on a 1 percent low-melting-point agarose slab gel (available from Bethesda Research Laboratories, Inc., USA) containing 1 $\mu$g/ml of ethidium bromide. The linear DNA of molecular weight 3.4 megadaltons with a break at one of the EcoRI sites was recovered from the gel plate and was melted in a water bath at 65°C after addition of two volumes of a buffer containing 40 mM Tris, 20 mM sodium acetate and 1 mM 2Na-EDTA (disodium ethylenediaminetetraacetate). The resultant preparation was allowed to cool at room temperature and was thereafter mixed with an equal volume of phenol, followed by vigorous shaking. The mixture was subjected to centrifugation at 15,000 rpm for 3 minutes at room temperature and the aqueous layer containing DNA was recovered therefrom. The DNA solution was mixed with an equal volume of phenol and the above described procedure was repeated to collect a DNA-containing aqueous solution for a second time. Two hundred $\mu$l of a buffer (pH 8.0) containing 50 mM Tris, 10 mM EDTA (ethylenediaminetetraacetic acid), and 100 mM NaCl was added to the DNA solution. The resultant mixture was mixed with two volumes of cold ethanol of -20°C and was chilled at -20°C for 30 minutes. After cooling, the mixture was centrifuged at 15,000 rpm at 0°C for 5 minutes and the precipitates of DNA were washed with ethanol of -20°C. The resultant mixture was centrifuged at 15,000 rpm at 0°C for 2 minutes and the supernatant ethanol was discarded by decantation, whereupon the residue of the ethanol in the DNA precipitates was evaporated completely. The DNA fragments thus obtained were dissolved in 5 $\mu$l of sterile water. To the resultant aqueous solution of DNA were added 2 $\mu$l of a 10-fold concentrated T4 DNA polymerase buffer (containing 670 mM Tris-HCl (pH 8.0), 67 mM $MgCl_2$ and 70 mM $\beta$-mercaptoethanol), 8 ul of sterile water, and 0.8 $\mu$l of 2.5 mM deoxyribonucleoside triphosphates (dATP, dGTP, dCTP and TTP), followed by further addition of 0.5 $\mu$l of 4 unit/$\mu$l T4 DNA polymerase. The mixture was incubated at 37°C for 15 minutes. Then, 200 ul of a solution containing 50 mM Tris, 10 mM EDTA and 100 mM NaCl and further phenol were added to the reaction mixture to inactivate the enzymes contained therein. DNA fragments were then recovered following the above described ethanol precipitation procedure. The DNA fragments thus obtained were dissolved in 20 $\mu$l of sterile water, to which were added 3 $\mu$l of 10 mM ATP, 3 $\mu$l of 100 mM dithiothreitol, and 3 $\mu$l of a buffer consisting of 660 mM Tris-HCl (pH 7.6) and 66 mM $MgCl_2$, and further 1 $\mu$l of 3 unit/$\mu$l T4 DNA ligase. The resultant preparation was incubated at 15°C for 3 hours, followed by addition of 120 $\mu$l of sterile water to give a total volume of 150 $\mu$l.

(2) Transformation of E. coli with the Plasmid

To 150 $\mu$l of the plasmid DNA (approximately 0.5 $\mu$g in weight) obtained by the procedure (1) were added an equal volume of competent cells of E. coli and the resultant preparation was allowed to stand at 0°C for 10 minutes. The cells of E. coli were grown in 0.7 ml of L-broth at 37°C for an hour. The culture was plated onto the surface of an agar plate which consisted of L-broth containing 1.5% agar and 20 $\mu$g/ml tetracycline and the cells were further grown overnight at 37°C. Four transformants which formed colonies on the agar plate were checked for the sizes of the plasmid retained by each of the clones.

(3) Extraction and Determination of Molecular Weight of Plasmid and Confirmation of Recognition and Cleavage Site for EcoRI

Each of the transformants obtained by the procedure (2) above was suspended in 0.2 ml of a solution consisting of 0.2 mg/ml lysozyme and 0.05 mg/ml ribonuclease (RNase). The resultant preparation, with addition of 0.2 ml of 0.2% dodecylsodiumsulfate (SDS) solution, was incubated at room temperature for 1 to 2 minutes, subsequently allowed to stand at 0°C for 10 minutes, and centrifuged at 20,000 rpm for 10 minutes at 0°C. To the supernatant thus obtained was added an equal volume of phenol saturated with buffer "A" which consisted of 10mM Tris and 0.1 mM EDTA (pH 7.4), followed by vigorous shaking. The resultant mixture was centrifuged at 15,000 rpm for 3 minutes at room temperature to collect the aqueous layer containing DNA. Fifteen microlitre fractions of the DNA-containing preparation were electrophoresed on a 0.8% agarose gel. Molecular weight of the DNA extracted from each of the four transformants was about 3.4 megadaltons.

Tests were then conducted to make certain that the lefthand one (Fig. 1) of its two EcoRI sites in the original plasmid is absent in the DNA extracted from some of the four transformants as had been expected. For this purpose, the DNA extracted from the transformants was further collected by the previously described ethanol precipitation procedure, from the remaining fractions of the DNA-containing aqueous layer. The collected DNA (plasmid DNA) was dissolved in 100 $\mu$l of the buffer "A". To 10 ul of the resultant solution of the plasmid DNA were added 10 ul of a 2-fold concentrated buffer (containing 20 mM Tris-HCl (pH 7.6), 14 mM MgCl$_2$, 14 mM $\beta$-mercaptoethanol and 100 mM NaCl) and 2 $\mu$l of 6 unit/$\mu$l EcoRI. The mixture was incubated at 37°C for 60 minutes and was heated at 70°C for 5 minutes to terminate the reaction. The total reaction mixture was electrophoresed on a 0.8% agarose gel, with the result that two groups of the DNA fragments produced by cleaving each plasmid (pHY340) at one of the EcoRI sites were collected. To 10 $\mu$l each of these two groups of DNA fragments were added 10 $\mu$l of a 2-fold concentrated buffer (containing 20 mM Tris-HCl (pH 7.6), 14 mM MgCl$_2$, 14 mM $\beta$-mercaptoethanol and 100 mM NaCl) and 2 $\mu$l each of 5 unit/$\mu$l SacII and 6 unit/$\mu$l EcoRI. The mixture was incubated at 37°C for 60 minutes. The reaction was terminated at 70°C in 5 minutes thereafter and the total reaction mixture was electrophoresed on a 0.8% agarose gel, with the result that a group of the plasmids which had been cut at the lefthand one of its EcoRI sites were collected as had been expected. This particular plasmid was named plasmid pHY339 (Fig. 1).

(4) Insertion of Hind III Linker

To 30 $\mu$l of the plasmid DNA pHY339 (approximately 0.1 $\mu$g/ml) were added 5 $\mu$l of a 10-fold concentrated buffer (containing 100 mM Tris-HCl (pH 7.6), 70 mM MgCl$_2$, 70 mM $\beta$-mercaptoethanol and 500 mM NaCl), and 5 $\mu$l of 8 unit/$\mu$l SacII. The mixture was incubated at 37°C for 60 minutes to digest the DNA molecules with the restriction enzyme SacII and was heated at 70°C for 5 minutes to terminate the reaction and was thereafter electrophoresed on a 1% low-melting-point agarose slab gel (containing 1 $\mu$g/ml of ethydium bromide) similar to that used in the procedure (1). The plasmid DNA fragments of molecular weight 3.4 megadaltons which were cut at the SacII site were extracted from the gel plate and the DNA fragments thus extracted were collected therefrom by phenol treatment and the cold ethanol precipitation procedure. The DNA fragments were dissolved in 20 $\mu$l of buffer "A". A 5 $\mu$l fraction of the resultant DNA solution was electrophoresed on a 0.8% agarose gel with the result that each of the plasmid DNA fragments was found to have a molecular weight of about 3.4 megadaltons. The 15 $\mu$l faction of the resultant DNA solution was treated with T4 DNA polymerase following the steps used in the procedure (1), whereupon DNA fragments were collected also by performing phenol treatment and the cold ethanol precipitation procedure. The collected DNA fragments were dissolved in 20 $\mu$l of buffer "A". To the resultant solution was added 1 $\mu$l of phosphorylated HindIII linker (dpCAAGCTTG; available from Takara Shuzo Co., Ltd., Japan; 0.01 optical density/ml). The resultant preparation was treated with T4 DNA ligase as in the procedure (1) to insert the linker to each of the DNA fragments and to circularize the DNA fragments. The solution was diluted with 120 $\mu$l of sterile water to give a total volume of 150 $\mu$l.

(5) Transformation of E. coli with the Plasmid

Following the steps of the procedure (2), transformation of E. coli was carried out with use of the 150 $\mu$l of DNA solution (approximately 1 $\mu$g) obtained as a result of the procedure (4).

(6) Extraction and Determination of Molecular Weight of Plasmid and Confirmation of Recognition and Cleavage Site for HindIII

Following the steps of the procedure (3) but using the transformants obtained by the procedure (5), the plasmid DNAs were extracted from several of the transformants for the estimations of their molecular weight. The result revealed that the DNA molecule at each plasmid had a molecular weight of about 3.4 megadaltons.

Tests were further conducted to make certain that the DNA molecule of each plasmid extracted from the transformants contains the HindIII site. For this purpose, 20 $\mu$l of the plasmid DNA thus extracted was added with 2 $\mu$l of a 10-fold concentrated buffer containing 100 mM Tris-HCl (pH 7.6), 70 mM MgCl$_2$, 70 mM $\beta$-mercaptoethanol and 500 mM NaCl, and 3 $\mu$l of 10 unit/$\mu$l HindIII. The mixture was incubated at 37°C for 90 minutes and was then heated at 70°C for 5 minutes to terminate the reaction. The total reaction mixture was electrophoresed on a 0.8% agarose gel, with the result that the original plasmid DNA was found to be able to be cut at the HindIII site. This particular plasmid was named pHY336.

(7) Insertion of Polylinker

i) Preparation of the polylinker

Plasmid $\pi$AN7 (a gift from Professor Saito's Office, Institute of Applied Microbiology, The University of Tokyo) was employed to prepare a polylinker having recognition and cleavage sites for the restriction enzymes in the sequence of

$$\underline{\text{EcoRI}} - \left\{ \begin{array}{c} \underline{\text{SmaI}} \\ \underline{\text{XmaI}} \end{array} \right\} - \underline{\text{BamHI}} - \left\{ \begin{array}{c} \underline{\text{SalI}} \\ \underline{\text{AccI}} \\ \underline{\text{HincII}} \end{array} \right\} - \underline{\text{PstI}} - \underline{\text{BglII}} - \underline{\text{XbaI}} - \underline{\text{HindIII}}.$$

To 20 $\mu$l of the plasmid AN7 (2 mg/ml) were added 3 $\mu$l of a 10-fold concentrated buffer (containing 100 mM Tris-HCl (pH 7.6), 70 mM MgCl$_2$, 70 mM $\beta$-mercaptoethanol and 500 mM NaCl), 5 $\mu$l of sterile water, and 3 $\mu$l of 10 unit/$\mu$l HindIII. The mixture was incubated at 37°C for 60 minutes to digest the plasmid with HindIII. Thereafter, 3 $\mu$l of 6 unit/$\mu$l EcoRI was added to the reaction mixture, followed by further incubation at 37°C for 60 minutes to further digest the plasmid with EcoRI. For the purpose of disintegrating into pieces each of the DNA fragments outside the polylinker region, 2 $\mu$l of 3.5 unit/$\mu$l HaeIII was added to the reaction mixture. The resultant preparation was incubated at 37°C for 60 minutes and the reaction was terminated at 70°C in 5 minutes thereafter. The reaction mixture was subjected to phenol treatment and ethanol precipitation procedure to collect the precipitates of DNA fragments following some of the steps of the procedure (1).

ii) Preparation of vector.

To 30 $\mu$l of plasmid pHY336 (approximately 0.1 $\mu$g/$\mu$l) were added 3.5 $\mu$l of a 10-fold concentrated buffer containing 100 mM Tris-HCl (pH 7.6), 70 mM MgCl$_2$, 70 mM $\beta$-mercaptoethanol and 500 mM NaCl, and 3 $\mu$l of 10 unit/$\mu$l HindIII. The mixture was incubated at 37°C for 60 minutes. To the reaction mixture, 3$\mu$l of 6 unit/$\mu$l EcoRI was added and further incubated at 37°C for 60 minutes and the reaction was terminated at 70°C in 5 minutes thereafter. The resultant reaction mixture was electrophoresed on a 0.8% agarose gel, with the result that the plasmid DNA fragments were found to have breaks at their HindIII and EcoRI sites. The DNA fragments were recovered by performing phenol treatment and an ethanol precipitation procedure also following some of the steps of the procedure (1).

iii) Insertion of polylinker into vector.

Each of the polylinker obtained by the steps i) and the vector prepared by the steps ii) above was dissolved in 10 ul of sterile water. The two solutions were then mixed together and thereafter the resultant solution was subjected to the ligation step of the procedure (1) to insert the polylinker into the vector, followed by addition of sterile water to give a total volume of 150 $\mu$l.

(8) Transformation of E. coli with the Plasmid

Following the steps of the procedure (2), transformation of E. coli was carried out with use of the polylinker-carrying DNA molecules (approximately 1 $\mu$g) obtained by the steps iii) of the procedure (7) above. The sizes of the plasmid DNA molecules carried on the transformants thus obtained were then determined.

(9) Extraction and Determination of Molecular Weight of the Plasmid and Confirmation of the Presence of Polylinker

Following the steps of the procedure (3) but using the transformants obtained by the procedure (8), molecular weight estimations were carried out on the plasmids extracted from 40 strains selected from the transformants. Out of those DNA plasmids, those which have revealed to have molecular weights of about 3.0 megadaltons were further selected for treatment with phenol, thus collecting DNA molecules as in the procedure (1).

These plasmids were then cleaved at their cleavage sites for the restriction enzymes EcoRI, SmaI, BamHI, SalI, PstI, BgIII, XbaI and HindIII to see if the polylinker derived from the plasmid AN7 had actually been inserted into each of the plasmids. For this purpose, the plasmid DNA obtained from the transformants were dissolved in 100 $\mu$l of buffer "A". The resultant preparation was divided into eight 10 $\mu$l fractions, to each of which were further added 10 $\mu$l of a 2-fold concentrated buffer containing 20 mM Tris-HCl (pH 7.6), 14 mM MgCl$_2$, 14 mM $\beta$-mercaptoethanol and 100 mM NaCl, and thereafter 1 $\mu$l of 6 unit/$\mu$l EcoRI, 4 unit/$\mu$l SmaI, 1 unit/$\mu$l BamHI, 8 unit/$\mu$l SalI, 5 unit/$\mu$l PstI, 4.5 unit/$\mu$l BgIII, 9 unit/$\mu$l XbaI, or 10 unit/$\mu$l HindIII. Each mixture was incubated at 37°C for 60 minutes. The reaction was terminated at 70°C in 5 minutes thereafter and the entire volume of each mixture was electrophoresed on a 0.8% agarose gel. It was found that all of the plasmid DNA contained in each mixture had breaks at the sites for EcoRI, SmaI, BamHI, SalI, PstI, BgIII, XbaI and HindIII, showing that the plasmid pHY336 carried an insert of the polylinker. The polylinker-carrying plasmid thus obtained was named pHY300PLK.

(10) Transformation of E. coli and B. subtilis with Plasmid pHY300PLK

To ascertain that the plasmid pHY300PLK is operable as a shuttle vector, transformation of E. coli and B. subtilis was performed with the DNA molecules extracted from the transformants used in the procedure (9) above. For reference purposes, plasmid pHY340 and plasmid pHY460 were used as control vectors, the latter being disclosed in United States Patent Application Serial No. 574,180, EPC Application No. 84-30-0403.7 and Japanese Patent Application No. 58-9740.

i) Transformation of E. coli

Hundred $\mu$l of competent cells of E. coli were added to 5 $\mu$l each of the plasmid DNA (approximately 1 $\mu$g) obtained in the course of the procedure (9), the plasmid pHY340 (approximately 1 $\mu$g) and the plasmid pHY460 (approximately 1 $\mu$g). Each resultant preparation was allowed to stand at 0°C for 15 minutes to enable the DNA to penetrate into the cells of E. coli. The cells of E. coli were poured with 0.9 ml of L-broth and was then grown at 37°C for an hour therein. Each culture thus obtained was plated onto an L-plate containing 1.5% agar and 20 $\mu$g/ml tetracycline and the cells were further grown overnight at 37°C.

ii) Transformation of B. subtilis

Five hundred $\mu$l of competent cells of B. subtilis were added to 10 $\mu$l each of the plasmid (approximately 1 $\mu$g) obtained in the course of the procedure (9), the plasmid pHY340 (approximately 1 $\mu$g) and the plasmid pHY460 (approximately 1 $\mu$g). The resultant preparations were cultured with shaking at 37°C for 90 minutes. The cultured preparation was plated onto the surface of an agar plate similar to the medium used in the steps i) above and the cells were further grown overnight at 37°C.

The numbers of the cells of E. coli and B. subtilis thus transformed per 1 $\mu$g each of the plasmids pHY300PLK, pHY340 and pHY460, and the ratio between such numbers are shown in the following table.

| Plasmid | pHY300PLK | pHY340 | pHY460 |
|---|---|---|---|
| E. coli | $8.3 \times 10^5$ | $9.5 \times 10^5$ | $8.3 \times 10^5$ |
| B. subtilis | $3.2 \times 10^5$ | $8.8 \times 10^4$ | $7.25 \times 10^4$ |
| B. subtilis/E. coli | 0.386 | 0.092 | 0.087 |

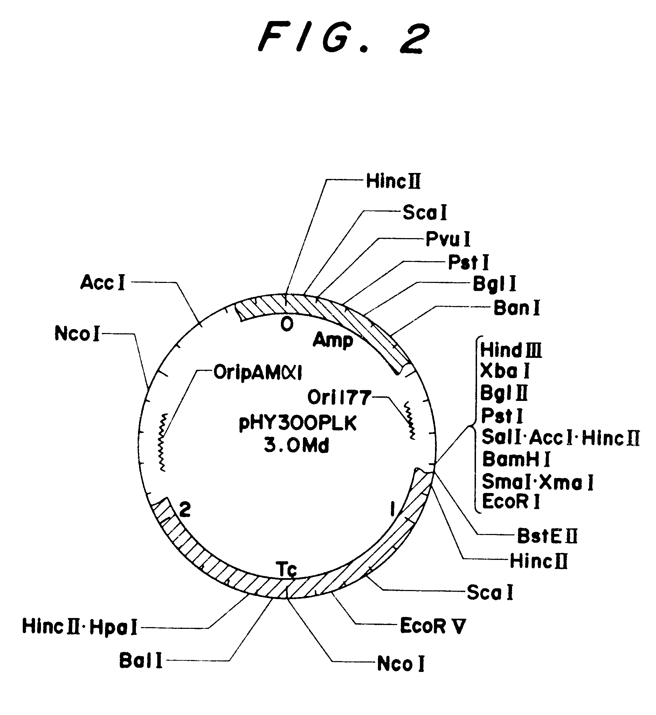

The results shown in the table above demonstrate that the plasmid obtained in the course of the

9

procedure (9) is a shuttle vector which reciprocates between E. coli and B. subtilis. This chimeric plasmid vector was named pHY300PLK, the cleavage map of which is shown in Fig. 2. The E. coli and B. subtilis transformed with this plasmid pHY300PLK have been deposited under the names of Escherichia coli C600 (pHY300PLK) and Bucillus subtilis (pHY300PLK) at Fermentation Research Institute, Agency of Industrial Science and Technology of Japan, as Deposition No. FERM BP-744 and FERM BP-753, respectively.

Example 2

Plasmid pHY301PLK was prepared, following the procedures of Example 1 with the exception that a polylinker (commercially available from Amersham (UK), Code No. M13mp8) having a sequence of recognition and cleavage sites for the following series of restriction enzymes:

$$\underline{EcoRI} - \left\{ \begin{array}{l} \underline{SmaI} \\ \underline{XmaI} \end{array} \right\} - \underline{BamHI} - \left\{ \begin{array}{l} \underline{SalI} \\ \underline{AccI} \\ \underline{HincII} \end{array} \right\} - \underline{PstI} - \underline{HindIII}.$$

It was ascertained that the plasmid pHY301PLK was also operable as a shuttle vector between E. coli and B. subtilis.

Example 3

Plasmid pHY302PLK was prepared, following the procedures of Example 1 with the exception that a polylinker (commercially available from Amersham, Code No. M13mp10) having a sequence of recognition and cleavage sites for the following series of restriction enzymes:

$$\underline{EcoRI} - \underline{SstI} - \left\{ \begin{array}{l} \underline{SmaI} \\ \underline{XmaI} \end{array} \right\} - \underline{BamHI} - \underline{XbaI} - \left\{ \begin{array}{l} \underline{SalI} \\ \underline{AccI} \\ \underline{HincII} \end{array} \right\} - \underline{PstI} - \underline{HindIII}$$

It was also ascertained that the plasmid pHY302PLK was operable as a shuttle vector between E. coli and B. subtilis.

**Claims**

1.  A chimeric plasmid vector useful as a shuttle vector capable of reciprocating between E. coli and B. subtilis, the vector containing (a) a tetracycline resistance gene region derived from the plasmid pAMα1 of Streptococcus faecalis, (b) an ampicillin resistance gene region derived from the vector pACYC177 of Escherichia coli, (c) a DNA replication origin derived from said plasmid pAMα1, (d) a DNA replication origin derived from said vector pACYC177, and (e) a polylinker region having a recognition and cleavage site for the restriction enzyme EcoRI at one terminal and a recognition and cleavage site for the restriction enzyme HindIII at the other terminal of the polylinker region, the plasmid vector having unique recognition and cleavage sites for the restriction enzymes BalI, HpaI and EcoRV, which sites are located in said tetracycline resistance gene region, and the plasmid vector having unique recognition and cleavage sites for the restriction enzymes PvuI, BglI and BanI, which sites are located in said ampicillin resistance gene region.

2.  A chimeric plasmid vector according to claim 1, denoted pHY300PLK herein, which has a molecular weight of about 3.0 megadaltons and whose restriction enzyme man is as shown in Fig. 2 of the drawings.

3.  A chimeric plasmid vector according to claim 1, denoted pHY301PLK herein, which has a molecular weight of about 3.0 megadaltons and whose restriction enzyme map is as shown in Fig. 3 of the drawings.

4.  A chimeric plasmid vector according to claim 1, denoted pHY302PLK herein, which has a molecular weight of about 3.0 megadaltons and whose restriction enzyme map is as shown in Fig. 4 of the drawings.

5. A chimeric plasmid vector according to claim 1 wherein the polylinker region is a DNA fragment having a sequence of recognition and cleavage sites for restriction enzymes sequenced in the form of

$$EcoRI - \begin{bmatrix} SmaI \\ \overline{XmaI} \end{bmatrix} - BamHI - \begin{bmatrix} SalI \\ \overline{AccI} \\ \overline{HincII} \end{bmatrix} - PstI - BglII - XbaI - HindIII$$

and a nucleotide sequence of

5'-GAATTCCCGGGGATCCGTCGACCTGCAGATCTCTAGAAGCTT-3' .

6. A chimeric plasmid vector according to claim 1 wherein the polylinker region is a DNA fragment having a sequence of recognition and cleavage sites for restriction enzymes sequenced in the form of

$$EcoRI - \begin{bmatrix} SmaI \\ \overline{XmaI} \end{bmatrix} - BamHI - \begin{bmatrix} SalI \\ \overline{AccI} \\ \overline{HincII} \end{bmatrix} - PstI - HindIII$$

and a nucleotide sequence of

5'GAATTCCCGGGGATCCGTCGACCTGCAGCCAAGCTT-3'.

7. A chimeric plasmid vector according to claim 1 wherein the polylinker region is a DNA fragment having a sequence of recognition and cleavage sites for restriction enzymes sequenced in the form of

$$EcoRI - SstI - \begin{bmatrix} SmaI \\ \overline{XmaI} \end{bmatrix} - BamHI - XbaI - \begin{bmatrix} SalI \\ \overline{AccI} \\ \overline{HincII} \end{bmatrix} - PstI - HindIII$$

and a nucleotide sequence of

5'-GAATTCGAGCTCGCCCGGGGATCCTCTAGAGTCGACCTGCAGCCCAAGCTT-3'.

8. A process for preparing a chimeric plasmid vector claimed in any one of the preceding claims, which process comprises:
   (A) enzymatically cleaving a chimeric plasmid containing:
      (a) a tetracycline resistance gene region derived from the plasmid pAMα1 of Streptococcus faecalis,
      (b) an ampicillin resistance gene region derived from the vector pACYC177 or Escherichia coli,
      (c) a DNA replication origin derived from said plasmid pAMα1, and
      (d) a DNA replication origin derived from said vector pACYC177,
   said tetracycline resistance gene region containing a unique site at which said chimeric plasmid is recognized and cleaved by the restriction enzyme Ball and said ampicillin resistance gene region containing a unique site at which said chimeric plasmid is recognized and cleaved by the restriction enzymes BgII and PstI,
   the enzymatic cleavage being such that the tetracycline resistance gene region (a) and the DNA replication origin (c) are retained in the DNA sequence derived from the plasmid pAMα1, and the ampicillin reistance gene region (b) and the DNA replication origin (d) are retained in the DNA sequence derived from the vector pACYC177; and
      (b) ligating to include the polylinker region.

9. A process for preparing a chimeric plasmid vector claimed in any one of claims 1-7, which process comprises excising the section of the plasmid pHY336 between the EcoRI and HindIII sites and substituting it by the polylinker region, which pHY336 has a molecular weight of about 3.4 megadaltons and whose restriction enzyme map is shown in Figure 1 of the drawings.

10. A process according to claim 9 wherein the plasmid pHY336 has been prepared by a process comprising:

(a) partially digesting plasmid pHY340 with EcoRI to obtain a fragment open at one of the two EcoRI sites of the plasmid pHY340, which has a molecular weight of 3.4 megadaltons and whose restriction enzyme map is shown in Figure 1 of the drawings, said fragment having a molecular weight of 3.4 megadaltons;

(b) treating the fragment with T4 DNA polymerase and T4 DNA ligase to produce a circular DNA;

(c) introducing the circular DNA into Escherichia coli and extracting plasmid pHY339 from the resultant transformant, the plasmid having unique EcoRI and SacII sites; and

(d) cleaving the plasmid pHY339 with SacII, treating the resultant linear DNA with T4 DNA polymerase, adding phosphorylated HindIII linker, and further treating with T4 DNA ligase, to produce plasmid pHY336 which has a HindIII site in place of the SacII site in the plasmid pHY339.

11. A method employing a vector to convey a gene into a host cell, to retain and express the gene therein, in which method there is used as vector a chimeric plasmid vector claimed in any one of claims 1-7 or prepared by a process claimed in any one of claims 8-10.

12. A microorganism, which microorganism is:    Escherichia coli C600 (pHY300PLK), or
        Bacillus subtilis (pHY300PLK),
as deposited at the Fermentation Research Institute, Agency of Industrial Science & Technology, Japan, under the respective deposition numbers of FERM BP-744 and FERM BP-753.

**Revendications**

1. Vecteur plasmidique chimérique utile comme vecteur-navette capable d'aller et venir entre *E. coli* et *B. subtilis*, le vecteur contenant (a) une région de gène de résistance à la tétracycline provenant du plasmide pAMα1 de *Streptococcus faecalis*, (b) une région de gène de résistance à l'ampicilline provenant du vecteur pACYC177 de *Escherichia coli*, (c) une origine de réplication d'ADN provenant dudit plasmide pAMα1, (d) une origine de réplication d'ADN provenant dudit vecteur pACYC177, et (e) une région de polyadaptateur ayant un site de reconnaissance et de clivage pour l'enzyme de restriction *Eco*RI à une extrémité et un site de clivage pour l'enzyme de restriction *Hind*III à l'autre extrémité de la région de polyadaptateur, le vecteur plasmidique ayant des sites de reconnaissance et de clivage uniques pour les enzymes de restriction *Bal*I, *Hpa*I et *Eco*RV, ces sites étant situés dans ladite région de gène de résistance à la tétracycline, et le vecteur plasmidique ayant des sites de reconnaissance et de clivage uniques pour les enzymes de restriction *Pvu*I, *Bgl*I et *Ban*I, ces sites étant situés dans ladite région de gène de résistance à l'ampicilline.

2. Vecteur plasmidique chimérique selon la revendication 1, dénommé ici pHY300PLK, qui a un poids moléculaire d'environ 3,0 mégadaltons et dont la carte de restriction enzymatique est telle que représentée sur la Figure 2 des dessins.

3. Vecteur plasmidique chimérique selon la revendication 1, dénommé ici pHY301PLK, qui a un poids moléculaire d'environ 3,0 mégadaltons et dont la carte de restriction enzymatique est telle que représentée sur la Figure 3 des dessins.

4. Vecteur plasmidique chimérique selon la revendication 1 dénommé ici pHY302PLK, qui a un poids moléculaire d'environ 3,0 mégadaltons et dont la carte de restriction enzymatique est telle que représentée sur la Figure 4 des dessins.

5. Vecteur plasmidique chimérique selon la revendication 1, dans lequel la région de polyadaptateur est un fragment d'ADN ayant une séquence de sites de reconnaissance et de clivage pour enzymes de restriction qui est ordonnèe sous la forme

$$EcoRI - \begin{bmatrix} SmaI \\ XmaI \end{bmatrix} - BamHI - \begin{bmatrix} SalI \\ AccI \\ HincII \end{bmatrix} - PstI - BglII - XbaI - HindIII$$

et une séquence nucléotidique

5'-GAATTCCCGGGGGATCCGTCGACCTGCAGATCTCTAGAAGCTT-3'.

6. Vecteur plasmidique chimérique selon la revendication 1, dans lequel la région de polyadaptateur est un fragment d'ADN ayant une séquence de sites de reconnaissance et de clivage pour enzymes de restriction qui est ordonnée sous la forme

$$EcoRI - \begin{bmatrix} SmaI \\ XmaI \end{bmatrix} - BamHI - \begin{bmatrix} SalI \\ AccI \\ HincII \end{bmatrix} - PstI - HindIII$$

et une séquence nucléotidique

5'-GAATTCCCGGGGGATCCGTCGACCTGCAGCCAAGCTT-3'.

7. Vecteur plasmidique chimérique selon la revendication 1, dans lequel la région de polyadaptateur est un fragment d'ADN ayant une séquence de sites de reconnaissance et de clivage pour enzymes de restriction qui est ordonnée sous la forme

$$EcoRI - SstI - \begin{bmatrix} SmaI \\ XmaI \end{bmatrix} - BamHI - XbaI - \begin{bmatrix} SalI \\ AccI \\ HincII \end{bmatrix} - PstI - HindIII$$

et une séquence nucléotidique

5'-GAATTCGAGCTCGCCCGGGGGATCCTCTAGAGTCGACCTGCAGCCCAAGCTT-3'.

8. Procédé de préparation d'un vecteur plasmidique chimérique revendiqué dans l'une quelconque des revendications précédentes, ce procédé consistant à :
   (A) cliver enzymatiquement un plasmide chimérique contenant :
   (a) une région de gène de résistance à la tétracycline provenant du plasmide pAMα1 de *Streptococcus faecalis,*
   (b) une région de gène de résistance à l'ampicilline provenant du vecteur pACYC177 de *Escherichia coli,*
   (c) une origine de réplication d'ADN provenant dudit plasmide pAMα1, et
   (d) une origine de réplication d'ADN provenant dudit vecteur pACYC177,
   ladite région de gène de résistance à la tétracycline contenant un site unique au niveau duquel ledit plasmide chimérique est reconnu et clivé par l'enzyme de restriction *Bal*I et ladite région de gène de résistance à l'ampicilline contenant un site unique au niveau duquel ledit plasmide chimérique est reconnu et clivé par les enzymes de restriction *Bgl*I et *Pst*I, le clivage enzymatique étant tel que la région de gène de résistance à la tétracycline (a) et l'origine de réplication d'ADN (c) sont retenues dans la séquence d'ADN provenant du plasmide pAMα1, et que la région de gène de résistance à l'ampicilline (b) et l'origine de réplication d'ADN (d) sont retenues dans la séquence d'ADN provenant du vecteur pACYC177 ; et
   (B) assembler par ligature de façon à inclure la région de polyadaptateur.

9. Procédé de préparation d'un vecteur plasmidique chimérique tel que revendiqué dans l'une quelconque des revendications 1 à 7, ce procédé consistant à exciser la section du plasmide pHY336 comprise entre les sites *Eco*RI et *Hind*III et la remplacer par la région de polyadaptateur, le plasmide pHY336 ayant un poids moléculaire d'environ 3,4 mégadaltons et ayant la carte de restriction enzymatique représentée sur la Figure 1 des dessins.

**10.** Procédé selon la revendication 9, dans lequel le plasmide pHY336 a été préparé par un procédé consistant à :

(a) digérer partiellement le plasmide pHY340 avec *Eco*RI pour obtenir un fragment ouvert à l'un des deux sites *Eco*RI du plasmide pHY340 qui a un poids moléculaire de 3,4 mégadaltons et dont la carte de restriction enzymatique est représentée sur la Figure 1 des dessins, ledit fragment ayant un poids moléculaire de 3,4 mégadaltons ;

(b) traiter le fragment avec l'ADN-polymérase de T4 et l'ADN-ligase de T4 pour produire un ADN circulaire ;

(c) introduire l'ADN circulaire dans *Escherichia coli* et extraire le plasmide pHY339 du transformant résultant, le plasmide ayant des sites *Eco*RI et *Sac*II uniques ; et

(d) cliver le plasmide pHY339 avec *Sac*II, traiter l'ADN linéaire résultant avec l'ADN-polymérase de T4, ajouter un adaptateur *Hin*dIII phosphorylé, et traiter encore avec l'ADN-ligase de T4, pour produire le plasmide pHY336 qui comporte un site *Hin*dIII à la place du site *Sac*II du plasmide pHY339.

**11.** Méthode faisant usage d'un vecteur pour transporter un gène dans une cellule-hôte afin de retenir et exprimer le gène dans celle-ci, méthode dans laquelle le vecteur utilisé est un vecteur plasmidique chimérique revendiqué dans l'une quelconque des revendications 1 à 7 ou préparé par un procédé revendiqué dans l'une quelconque des revendications 8 à 10.

**12.** Micro-organisme, ce micro-organisme étant :

*Escherichia coli* C600 (pHY300PLK), ou

*Bacillus subtilis* (pHY300PLK),

tels que déposés à l'Institut de Recherche sur les Fermentations, Agence de la Science et de la Technologie Industrielles, Japon, sous les numéros de dépôt respectifs FERM BP-744 et FERM BP-753.

**Patentansprüche**

**1.** Chimärer Plasmidvektor, welcher als Shuttle-Vektor brauchbar und imstande ist, sich zwischen E. coli und B. subtilis hin- und herzubewegen, wobei der Vektor

(a) einen Tetracyclinresistenz-Genbereich, der aus dem Plasmid pAMα1 von Streptococcus faecalis stammt,

(b) einen Ampicillinresistenz-Genbereich, der aus dem Vektor pACYC177 von Escherichia coli stammt,

(c) einen den DNA-Replikationsursprung, der aus dem Plasmid pAMα1 stammt,

(d) einen DNA-Replikationsursprung, der aus dem Vektor pACYC177 stammt, und

(e) einen Polylinkerbereich, der eine Erkennungs- und Schnittstelle für das Restriktionsenzym EcoRI an einem Ende und eine Erkennungs- und Schnittstelle für das Restriktionsenzym HindIII an dem anderen Ende des Polylinkerbereichs aufweist,

enthält, und der Plasmidvektor ferner einzige, ganz bestimmte Erkennungs- und Schnittstellen für die Restriktionsenzyme BalI, HpaI und EcoRV enthält, wobei diese Schnittstellen im Tetracyclinresistenz-Genbereich liegen, und daß der Plasmidvektor einzige, ganz bestimmte Erkennungs- und Schnittstellen für die Restriktionsenzyme PvuI, BglI und BanI enthält, wobei diese Schnittstellen im Ampicillinresistenz-Genbereich liegen.

**2.** Chimärer Plasmidvektor gemäß Anspruch 1, bezeichnet als pHY300PLK, welcher ein Molekulargewicht von etwa 3,0 Megadalton besitzt und dessen Restriktionsenzymkarte in Abbildung 2 der Zeichnungen gezeigt wird.

**3.** Chimärer Plasmidvektor gemäß Anspruch 1, bezeichnet als PHY301PLK, welcher ein Molekulargewicht von etwa 3,0 Megadalton besitzt und dessen Restriktionsenzymkarte in Abbildung 3 der Zeichnungen gezeigt wird.

**4.** Chimärer Plasmidvektor gemäß Anspruch 1, bezeichnet als pHY302PLK, welcher ein Molekulargewicht von etwa 3,0 Megadalton besitzt und dessen Restriktionsenzymkarte in Abbildung 4 der Zeichnungen

14

gezeigt wird.

5. Chimärer Plasmidvektor gemäß Anspruch 1 bei dem der Polylinkerbereich ein DNA-Fragment ist, welches eine Folge von Erkennungs- und Schnittstellen für Restriktionsenzyme, sequenziert als

$$\underline{\text{Eco}}\text{RI} \quad - \begin{bmatrix} \underline{\text{Sma}}\text{I} \\ \underline{\text{Xma}}\text{I} \end{bmatrix} - \underline{\text{Bam}}\text{HI} \quad - \begin{bmatrix} \underline{\text{Sal}}\text{I} \\ \underline{\text{Acc}}\text{I} \\ \underline{\text{Hinc}}\text{II} \end{bmatrix} - \underline{\text{Pst}}\text{I} \quad - \underline{\text{Bgl}}\text{II} \quad - \underline{\text{Xba}}\text{I} - \underline{\text{Hind}}\text{III} ,$$

und eine Nukleotidsequenz von

5'-GAATTCCCGGGGGATCCGTCGACCTGCAGATCTCTAGAAGCTT-3'

enthält.

6. Chimärer Plasmidvektor gemäß Anspruch 1, bei dem der Polylinkerbereich ein DNA-Fragment ist, welches eine Folge von Erkennungs- und Schnittstellen für Restriktionsenzyme, sequenziert als

$$\underline{\text{Eco}}\text{RI} \quad - \begin{bmatrix} \underline{\text{Sma}}\text{I} \\ \underline{\text{Xma}}\text{I} \end{bmatrix} - \underline{\text{Bam}}\text{HI} \quad - \begin{bmatrix} \underline{\text{Sal}}\text{I} \\ \underline{\text{Acc}}\text{I} \\ \underline{\text{Hinc}}\text{II} \end{bmatrix} - \underline{\text{Pst}}\text{I} \quad - \underline{\text{Hind}}\text{III} ,$$

und eine Nukleotidsequenz von

5'-GAATTCCCGGGGGATCCGTCGACCTGCAGCCAAGCTT-3'

enthält.

7. Chimärer Plasmidvektor gemäß Anspruch 1, bei dem der Polylinkerbereich ein DNA-Fragment ist, welches eine Folge von Erkennungs- und Schnittstellen für Restriktionsenzyme, sequenziert als

$$\underline{\text{Eco}}\text{RI} - \underline{\text{Sst}}\text{I} \quad - \begin{bmatrix} \underline{\text{Sma}}\text{I} \\ \underline{\text{Xma}}\text{I} \end{bmatrix} - \underline{\text{Bam}}\text{HI} - \underline{\text{Xba}}\text{I} - \begin{bmatrix} \underline{\text{Sal}}\text{I} \\ \underline{\text{Acc}}\text{I} \\ \underline{\text{Hinc}}\text{II} \end{bmatrix} - \underline{\text{Pst}}\text{I} \quad - \underline{\text{Hind}}\text{III} ,$$

und eine Nukleotidsequenz von

5'-GAATTCGAGCTCGCCCGGGGATCCTCTAGAGTCGACCTGCAGCCCAAGCTT-3'

enthält.

8. Verfahren zur Herstellung eines chimären Plasmidvektors nach einem der vorhergehenden Ansprüche, wobei das Verfahren umfaßt:

    (A) Enzymatische Spaltung eines chimären Plasmids, das

        (a) einen Tetracyclinresistenz-Genbereich, der aus dem Plasmid pAMα1 von <u>Streptococcus faecalis</u> stammt,

        (b) einen Ampicillinresistenz-Genbereich, der aus dem Vektor pACYC177 von <u>Escherichia coli</u> stammt,

        (c) einen DNA-Replikationsursprung, der von dem Plasmid pAMα1 stammt und

        (d) einen DNA-Replikationsursprung, der von dem Vektor pACYC177 stammt,

    enthält,

    wobei der Tetracyclinresistenz-Genbereich eine einzige, ganz bestimmte Stelle enthält, an der das chimäre Plasmid durch das Restriktionsenzym Ball erkannt und geschnitten wird, und der Amplicillinresistenz-Genbereich eine einzige, ganz bestimmte Stelle enthält, an der das chimäre Plasmid durch die Restriktionsenzyme Bgll und Pstl erkannt und geschnitten wird,

wobei die enzymatische Spaltung derart ist, daß der Tetracyclinresistenz-Genbereich (a) und der DNA-Replikationsursprung (c) in dem DNA-Fragment verbleiben, das von dem Plasmid pAMα1 stammt, und der Amplicillinresistenz-Genbereich (b) und der DNA-Replikationsursprung (d) in dem DNA-Fragment verbleiben, das von dem Vektor pACYC177 stammt, und

(b) Ligation, um den Polylinkerbereich einzuschließen.

9. Verfahren zur Herstellung eines chimären Plasmidvektors gemäß einem der Ansprüche 1 bis 7, wobei das Verfahren das Herausschneiden des Abschnitts des Plasmids pHY336 zwischen den EcoRI und HindIII Schnittstellen und dessen Substitution durch einen Polylinkerbereich umfaßt, wobei pHY336 ein Molekulargewicht von etwa 3,4 Megadalton aufweist und dessen Restriktionsenzymkarte in Abbildung 1 der Zeichnungen gezeigt wird.

10. Verfahren gemäß Anspruch 9, bei dem das Plasmid pHY336 durch ein Verfahren hergestellt wurde, das umfaßt:

(a) teilweise Verdauung des Plasmids pHY340 mit EcoRI, um ein Fragment zu erhalten, das an einer der beiden EcoRI-Stellen des Plasmids pHY340, das ein Molekulargewicht von 3,4 Megadalton besitzt und dessen Restriktionsenzymkarte in Abbildung 1 der Zeichnungen gezeigt wird, geöffnet ist wobei das Fragment ein Molekulargewicht von 3,4 Megadalton besitzt;

(b) Behandlung des Fragments mit T4 DNA-Polymerase und T4 DNA-Ligase, um zirkuläre DNA herzustellen;

(c) Einbringen der zirkulären DNA in Escherichia coli und Extraktion des Plasmids pHY339 aus der resultierenden Transformanten, wobei das Plasmid einzige, ganz bestimmte EcoRI- und SacII-Schnittstellen aufweist; und

(d) Spaltung des Plasmids pHY339 mit SacII, Behandlung der resultierenden linearen DNA mit T4 DNA-Polymerase, Zugabe phosphorylierten HindIII-Linkers und Weiterbehandlung mit T4 DNA-Ligase, um das Plasmid pHY336 herzustellen, das eine HindIII-Schnittstelle anstelle der SacII-Schnittstelle im Plasmid pHY339 enthält.

11. Verfahren unter Verwendung eines Vektors, um Gene in Gastzellen einzubringen, um die Gene darin zu bewahren und zu exprimieren, wobei bei diesem Verfahren als Vektoren chimäre Plasmidvektoren gemäß einem der Ansprüche 1 bis 7 oder hergestellt nach einem Verfahren gemäß einem der Ansprüche 8 bis 10 verwendet werden.

12. Mikroorganismus, bei dem es sich handelt um:
Escherichia coli C600 (pHY300PLK), oder
Bacillus subtilis (pHY300PLK),
wie sie beim Fermentation Research Institute, Agency of Industrial Science & Technology, Japan, unter den jeweiligen Hinterlegungsnummern FERM BP-744 und FERM BP-753 hinterlegt wurden.

# FIG. 1

# FIG. 2

# FIG. 3

# F I G. 4